# EUROPEAN PATENT SPECIFICATION

(11) **EP 3 030 309 B1**
(45) Date of publication and mention of the grant of the patent: **10.04.2019**
(21) Application number: 14750969.9
(22) Date of filing: 05.08.2014
(51) Int. Cl.: A61N 1/05

(54) **SYSTEMS USING SEGMENTED TIP ELECTRODES FOR LEADS OF ELECTRICAL SIMULATION SYSTEMS**
SYSTEME ZUR VERWENDUNG VON SEGMENTIERTEN SPITZENELEKTRODEN FÜR ELEKTRISCHE STIMULATIONSSYSTEME
SYSTÈMES D'UTILISATION D'ÉLECTRODES À POINTE SEGMENTÉES POUR FILS DE SYSTÈMES DE STIMULATION ÉLECTRIQUE

(30) Priority: 07.08.2013 US 201361863132 P
(43) Date of publication of application: 15.06.2016
(73) Proprietor: Boston Scientific Neuromodulation Corporation, Valencia, CA 91355 (US)
(72) Inventor: HOWARD, Joshua Dale, Sacramento, CA 95817 (US)
(74) Representative: Vossius & Partner Patentanwälte Rechtsanwälte mbB
(86) International application number: PCT/US2014/049823
(87) International publication number: WO 2015/021069

(56) References cited:
- WO-A1-2009/025816
- US-A- 4 844 099
- US-A1- 2012 071 949

## Description

### FIELD

The present disclosure is directed to the area of implantable electrical stimulation systems and methods of making and using the systems. The present disclosure is also directed implantable electrical stimulation systems with leads having segmented tip electrodes, as well as methods of making and using the leads, segmented tip electrodes, and electrical stimulation systems.

### BACKGROUND

Electrical stimulation can be useful for treating a variety of conditions. Deep brain stimulation can be useful for treating, for example, Parkinson's disease, dystonia, essential tremor, chronic pain, Huntington's disease, levodopa-induced dyskinesias and rigidity, bradykinesia, epilepsy and seizures, eating disorders, and mood disorders. Typically, a lead with a stimulating electrode at or near a tip of the lead provides the stimulation to target neurons in the brain. Magnetic resonance imaging ("MRI") or computerized tomography ("CT") scans can provide a starting point for determining where the stimulating electrode should be positioned to provide the desired stimulus to the target neurons.

After the lead is implanted into a patient's brain, electrical stimulus current can be delivered through selected electrodes on the lead to stimulate target neurons in the brain. Typically, the electrodes are formed into rings disposed on a distal portion of the lead. The stimulus current projects from the ring electrodes equally in every direction. Because of the ring shape of these electrodes, the stimulus current cannot be directed to one or more specific positions around the ring electrode (*e.g.,* on one or more sides, or points, around the lead). Consequently, undirected stimulation may result in unwanted stimulation of neighboring neural tissue, potentially resulting in undesired side effects. Document WO-A-2009/025816 discloses the most relevant prior art.

### BRIEF SUMMARY

In one embodiment, an implantable electrical stimulation lead includes a lead body having a proximal end portion, a distal end portion, a distal tip, a longitudinal length, and a longitudinal surface. A set of segmented tip electrodes are disposed circumferentially about the distal tip of the lead body and are electrically-isolated from each other. Each segmented tip electrode of the set of segmented tip electrodes has an inner surface and an opposing outer stimulating surface exposed along the longitudinal surface of the lead body. A portion of the lead body is disposed against the inner surfaces of each of the segmented tip electrodes and circumferentially between each of the segmented tip electrodes. At least one non-tip electrode is disposed along the distal end portion of the lead body proximal to the set of segmented tip electrodes. Terminals are disposed along the proximal end portion of the lead body. Conductors electrically couple the terminals to the segmented tip electrodes and at least one non-tip electrode.

In another embodiment, a method of making a stimulation lead includes disposing a pre-tip-electrode along a distal tip of a lead body. The pre-tip-electrode includes a pre-tip-electrode body having an outer surface, an inner surface opposite the outer surface, a proximal end, and a distal end. The pre-tip-electrode body includes a ring-shaped base portion disposed along the proximal end of the pre-tip-electrode body; and a plurality of circumferentially-spaced-apart segmented tip electrodes coupled to the ring-shaped base portion and extending distally therefrom. The segmented tip electrodes each curve inwardly as they extend distally from the ring-shaped base portion such that the pre-tip-electrode body forms a substantially dome-shaped structure. The plurality of circumferentially-spaced-apart segmented tip electrodes are coupled to a plurality of terminals disposed along a proximal end portion of the lead body. Non-conductive material is placed against the inner surfaces of the pre-tip-electrode body and between the circumferentially-spaced-apart segmented tip electrodes to facilitate retention of the pre-tip-electrode with the lead body.

In yet another embodiment, a pre-tip-electrode for an electrical stimulation lead includes a pre-tip-electrode body having an outer surface, an inner surface opposite the outer surface, a proximal end, and a distal end. The pre-tip-electrode body includes a ring-shaped base portion disposed along the proximal end of the pre-tip-electrode body; and a plurality of circumferentially-spaced-apart segmented tip electrodes coupled to the ring-shaped base portion and extending distally therefrom. The segmented tip electrodes each curve inwardly as they extend distally from the ring-shaped base portion such that the pre-tip-electrode body forms a substantially dome-shaped structure. The invention is defined in claim 1. Any embodiment which is in contradiction to the subject-matter of claim 1 is not part of the invention.

### BRIEF DESCRIPTION OF THE DRAWINGS

Non-limiting and non-exhaustive embodiments of the present disclosure are described with reference to the following drawings. In the drawings, like reference numerals refer to like parts throughout the various figures unless otherwise specified.

For a better understanding of the present disclosure reference will be made to the following Detailed Description, which is to be read in association with the accompanying drawings, wherein:
FIG. 1 is a schematic side view of one embodiment of a device for brain stimulation, according to the disclosure;
FIG. 2 is a schematic diagram of radial current steering along various electrode levels along the length of a lead, according to the disclosure
FIG. 3A is a perspective view of an embodiment of a portion of a lead having a plurality of segmented electrodes, according to the disclosure;
FIG. 3B is a perspective view of a second embodiment of a portion of a lead having a plurality of segmented electrodes, according to the disclosure;
FIG. 3C is a perspective view of a third embodiment of a portion of a lead having a plurality of segmented electrodes, according to the disclosure;
FIG. 3D is a perspective view of a fourth embodiment of a portion of a lead having a plurality of segmented electrodes, according to the disclosure;
FIG. 3E is a perspective view of a fifth embodiment of a portion of a lead having a plurality of segmented electrodes, according to the disclosure;
FIG. 3F is a perspective view of a sixth embodiment of a portion of a lead having a plurality of segmented electrodes, according to the disclosure;
FIG. 3G is a perspective view of a seventh embodiment of a portion of a lead having a plurality of segmented electrodes, according to the disclosure;
FIG. 4 is a schematic side view of one embodiment of a distal end portion and a proximal end portion of a lead body, the lead body having a segmented tip electrode and several non-tip electrodes disposed along the distal end portion, and several terminals disposed along the proximal end portion, according to the invention;
FIG. 5A is a schematic perspective view of one embodiment of a pre-tip-electrode suitable for use in forming a set of segmented tip electrodes, the pre-tip-electrode including a base portion and multiple segmented tip electrodes attached to the base portion, according to the invention;
FIG. 5B is a schematic end view of one embodiment of the pre-tip-electrode of FIG. 5A, according to the invention;
FIG. 6A is a schematic end view of one embodiment of a set of segmented tip electrodes formed from the pre-tip-electrode of FIGS. 5A-5B, according to the invention;
FIG. 6B is a schematic perspective view of one embodiment of the set of segmented tip electrodes of FIG. 6A, according to the invention;
FIG. 7A is a schematic perspective view of one embodiment of a pre-tip-electrode, the pre-tip-electrode including a channel and multiple longitudinal grooves defined along an inner surface of the pre-tip-electrode, according to the invention;
FIG. 7B is a schematic longitudinal cross-sectional view of one embodiment of the pre-tip-electrode of FIG. 7A, according to the invention;
FIG. 8A is a schematic perspective view of another embodiment of a pre-tip-electrode suitable for use in forming a set of segmented tip electrodes, the pre-tip-electrode including a base portion and multiple segmented tip electrodes attached to the base portion, according to the invention;
FIG. 8B is a schematic distal perspective view of one embodiment of a set of segmented tip electrodes formed from the pre-tip-electrode of FIG. 8A, according to the invention;
FIG. 8C is a schematic proximal perspective view of one embodiment of the set of segmented tip electrodes of FIG. 8B, according to the invention; and
FIG. 8D is a schematic perspective side view of one embodiment of a segmented tip electrode of the set of segmented tip electrodes of FIGS. 8B-8C, according to the invention.

### DETAILED DESCRIPTION

The present disclosure is directed to the area of implantable electrical stimulation systems and methods of making and using the systems. The present disclosure is also directed implantable electrical stimulation systems with leads having segmented tip electrodes, as well as methods of making and using the leads, segmented tip electrodes, and electrical stimulation systems.

A lead for deep brain stimulation may include stimulation electrodes, recording electrodes, or a combination of both. At least some of the stimulation electrodes, recording electrodes, or both are provided in the form of segmented electrodes that extend only partially around the circumference of the lead. These segmented electrodes can be provided in sets of electrodes, with each set having electrodes radially distributed about the lead at a particular longitudinal position. For illustrative purposes, the leads are described herein relative to use for deep brain stimulation, but it will be understood that any of the leads can be used for applications other than deep brain stimulation, including spinal cord stimulation, peripheral nerve stimulation, or stimulation of other nerves and tissues.

Suitable implantable electrical stimulation systems include, but are not limited to, a least one lead with one or more electrodes disposed on a distal end of the lead and one or more terminals disposed on one or more proximal ends of the lead. Leads include, for example, percutaneous leads. Examples of electrical stimulation systems with leads are found in, for example, U.S. Patents Nos. 6,181,969; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,175,710; 8,224,450; 8,271,094; 8,295,944; 8,364,278; and 8,391,985; U.S. Patent Applications Publication Nos. 2007/0150036; 2009/0187222; 2009/0276021; 2010/0076535; 2010/0268298; 2011/0005069; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; and U.S. Patent Applications Serial Nos. 12/177,823; 13/667,953; and 13/750,725.

In at least some embodiments, a practitioner may determine the position of the target neurons using recording electrode(s) and then position the stimulation electrode(s) accordingly. In some embodiments, the same electrodes can be used for both recording and stimulation. In some embodiments, separate leads can be used; one with recording electrodes which identify target neurons, and a second lead with stimulation electrodes that replaces the first after target neuron identification. In some embodiments, the same lead may include both recording electrodes and stimulation electrodes or electrodes may be used for both recording and stimulation.

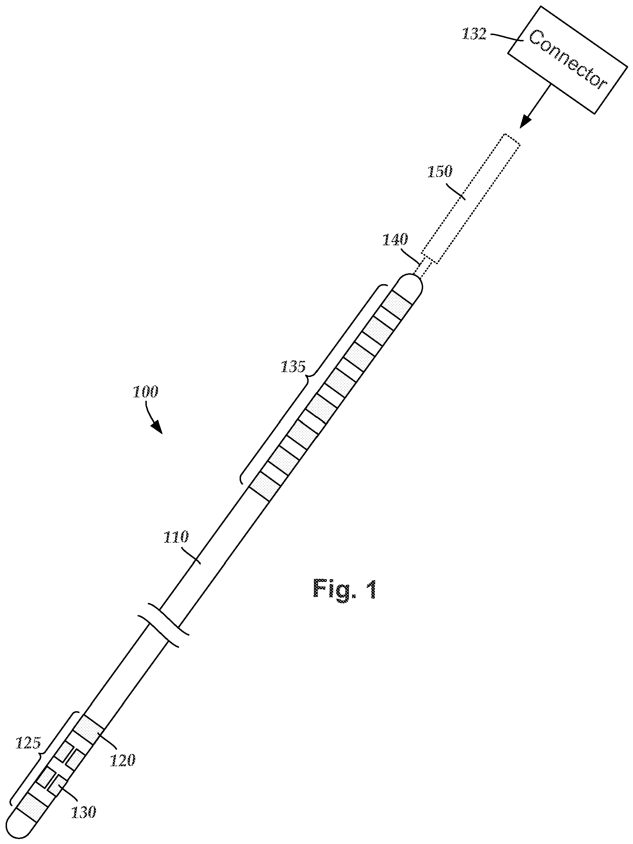

Figure 1 illustrates one embodiment of a device 100 for brain stimulation. The device includes a lead 110, a plurality of electrodes 125 disposed at least partially about a circumference of the lead 110, a plurality of terminals 135, a connector 132 for connection of the electrodes to a control unit, and a stylet 140 for assisting in insertion and positioning of the lead in the patient's brain. The stylet 140 can be made of a rigid material. Examples of suitable materials for the stylet include, but are not limited to, tungsten, stainless steel, and plastic. The stylet 140 may have a handle 150 to assist insertion into the lead 110, as well as rotation of the stylet 140 and lead 110. The connector 132 fits over a proximal end of the lead 110, preferably after removal of the stylet 140.

The control unit (not shown) is typically an implantable pulse generator that can be implanted into a patient's body, for example, below the patient's clavicle area. The pulse generator can have eight stimulation channels which may be independently programmable to control the magnitude of the current stimulus from each channel. In some cases the pulse generator may have more or fewer than eight stimulation channels (e.g., 4-, 6-, 16-, 32-, or more stimulation channels). The control unit may have one, two, three, four, or more connector ports, for receiving the plurality of terminals 135 at the proximal end of the lead 110.

In one example of operation, access to the desired position in the brain can be accomplished by drilling a hole in the patient's skull or cranium with a cranial drill (commonly referred to as a burr), and coagulating and incising the dura mater, or brain covering. The lead 110 can be inserted into the cranium and brain tissue with the assistance of the stylet 140. The lead 110 can be guided to the target location within the brain using, for example, a stereotactic frame and a microdrive motor system. In some embodiments, the microdrive motor system can be fully or partially automatic. The microdrive motor system may be configured to perform one or more the following actions (alone or in combination): insert the lead 110, retract the lead 110, or rotate the lead 110.

In some embodiments, measurement devices coupled to the muscles or other tissues stimulated by the target neurons, or a unit responsive to the patient or clinician, can be coupled to the control unit or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in tremor frequency or amplitude in response to stimulation of neurons. Alternatively, the patient or clinician may observe the muscle and provide feedback.

The lead 110 for deep brain stimulation can include stimulation electrodes, recording electrodes, or both. In at least some embodiments, the lead 110 is rotatable so that the stimulation electrodes can be aligned with the target neurons after the neurons have been located using the recording electrodes.

Stimulation electrodes may be disposed on the circumference of the lead 110 to stimulate the target neurons. Stimulation electrodes may be ring-shaped so that current projects from each electrode equally in every direction from the position of the electrode along a length of the lead 110. Ring electrodes typically do not enable stimulus current to be directed from only a limited angular range around of the lead. Segmented electrodes, however, can be used to direct stimulus current to a selected angular range around the lead. When segmented electrodes are used in conjunction with an implantable pulse generator that delivers constant current stimulus, current steering can be achieved to more precisely deliver the stimulus to a position around an axis of the lead (*i.e*., radial positioning around the axis of the lead).

To achieve current steering, segmented electrodes can be utilized in addition to, or as an alternative to, ring electrodes. Though the following description discusses stimulation electrodes, it will be understood that all configurations of the stimulation electrodes discussed may be utilized in arranging recording electrodes as well.

The lead 100 includes a lead body 110, one or more optional ring electrodes 120, and a plurality of sets of segmented electrodes 130. The lead body 110 can be formed of a biocompatible, non-conducting material such as, for example, a polymeric material. Suitable polymeric materials include, but are not limited to, silicone, polyurethane, polyurea, polyurethane-urea, polyethylene, or the like. Once implanted in the body, the lead 100 may be in contact with body tissue for extended periods of time. In at least some embodiments, the lead 100 has a cross-sectional diameter of no more than 1.5 mm and may be in the range of 0.5 to 1.5 mm. In at least some embodiments, the lead 100 has a length of at least 10 cm and the length of the lead 100 may be in the range of 10 to 70 cm.

The electrodes may be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use.

Each of the electrodes can either be used or unused (OFF). When the electrode is used, the electrode can be used as an anode or cathode and carry anodic or cathodic current. In some instances, an electrode might be an anode for a period of time and a cathode for a period of time.

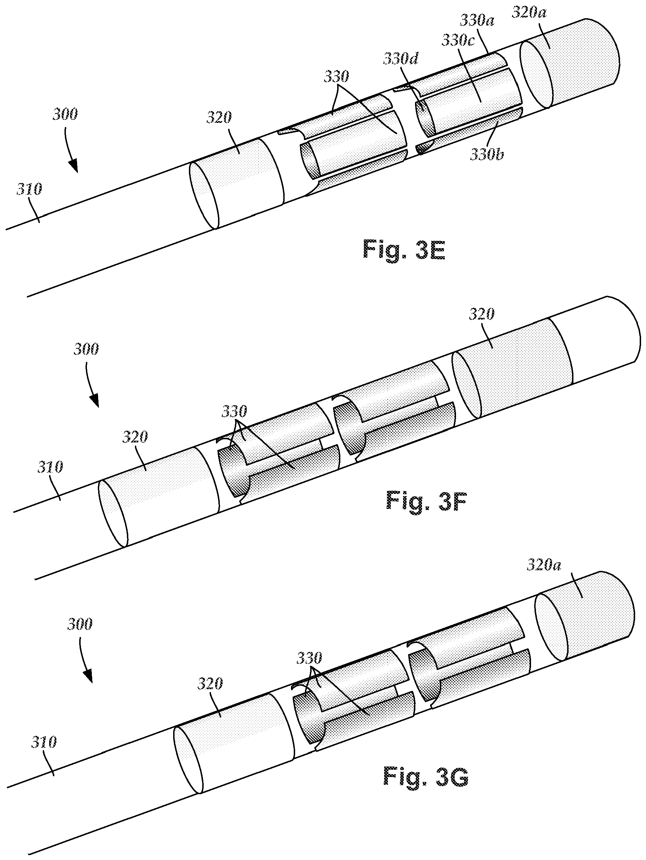

Stimulation electrodes in the form of ring electrodes 120 may be disposed on any part of the lead body 110, usually near a distal end of the lead 100. In Figure 1, the lead 100 includes two ring electrodes 120. Any number of ring electrodes 120 may be disposed along the length of the lead body 110 including, for example, one, two three, four, five, six, seven, eight, nine, ten, eleven, twelve, thirteen, fourteen, fifteen, sixteen or more ring electrodes 120. It will be understood that any number of ring electrodes may be disposed along the length of the lead body 110. In some embodiments, the ring electrodes 120 are substantially cylindrical and wrap around the entire circumference of the lead body 110. In some embodiments, the outer diameters of the ring electrodes 120 are substantially equal to the outer diameter of the lead body 110. The length of the ring electrodes 120 may vary according to the desired treatment and the location of the target neurons. In some embodiments the length of the ring electrodes 120 are less than or equal to the diameters of the ring electrodes 120. In other embodiments, the lengths of the ring electrodes 120 are greater than the diameters of the ring electrodes 120. The distal-most ring electrode 120 may be a tip electrode (see, e.g., tip electrode 320a of Figure 3E) which covers most, or all, of the distal tip of the lead.

Deep brain stimulation leads may include one or more sets of segmented electrodes. Segmented electrodes may provide for superior current steering than ring electrodes because target structures in deep brain stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue. Examples of leads with segmented electrodes include U.S. Patent Application Publication Nos. 2010/0268298; 2011/0005069; 2011/0130803; 2011/0130816; 2011/0130817; 2011/0130818; 2011/0078900; 2011/0238129; 2012/0016378; 2012/0046710; 2012/0071949; 2012/0165911; 2012/197375; 2012/0203316; 2012/0203320; 2012/0203321.

In Figure 1, the lead 100 is shown having a plurality of segmented electrodes 130. Any number of segmented electrodes 130 may be disposed on the lead body 110 including, for example, one, two three, four, five, six, seven, eight, nine, ten, eleven, twelve, thirteen, fourteen, fifteen, sixteen or more segmented electrodes 130. It will be understood that any number of segmented electrodes 130 may be disposed along the length of the lead body 110. A segmented electrode 130 typically extends only 75%, 67%, 60%, 50%, 40%, 33%, 25%, 20%, 17%, 15%, or less around the circumference of the lead.

The segmented electrodes 130 may be grouped into sets of segmented electrodes, where each set is disposed around a circumference of the lead 100 at a particular longitudinal portion of the lead 100. The lead 100 may have any number segmented electrodes 130 in a given set of segmented electrodes. The lead 100 may have one, two, three, four, five, six, seven, eight, or more segmented electrodes 130 in a given set. In at least some embodiments, each set of segmented electrodes 130 of the lead 100 contains the same number of segmented electrodes 130. The segmented electrodes 130 disposed on the lead 100 may include a different number of electrodes than at least one other set of segmented electrodes 130 disposed on the lead 100.

The segmented electrodes 130 may vary in size and shape. In some embodiments, the segmented electrodes 130 are all of the same size, shape, diameter, width or area or any combination thereof. In some embodiments, the segmented electrodes 130 of each circumferential set (or even all segmented electrodes disposed on the lead 100) may be identical in size and shape.

Each set of segmented electrodes 130 may be disposed around the circumference of the lead body 110 to form a substantially cylindrical shape around the lead body 110. The spacing between individual electrodes of a given set of the segmented electrodes may be the same, or different from, the spacing between individual electrodes of another set of segmented electrodes on the lead 100. In at least some embodiments, equal spaces, gaps or cutouts are disposed between each segmented electrode 130 around the circumference of the lead body 110. In other embodiments, the spaces, gaps or cutouts between the segmented electrodes 130 may differ in size or shape. In other embodiments, the spaces, gaps, or cutouts between segmented electrodes 130 may be uniform for a particular set of the segmented electrodes 130, or for all sets of the segmented electrodes 130. The sets of segmented electrodes 130 may be positioned in irregular or regular intervals along a length the lead body 110.

Conductor wires that attach to the ring electrodes 120 or segmented electrodes 130 extend along the lead body 110. These conductor wires may extend through the material of the lead 100 or along one or more lumens defined by the lead 100, or both. The conductor wires are presented at a connector (via terminals) for coupling of the electrodes 120, 130 to a control unit (not shown).

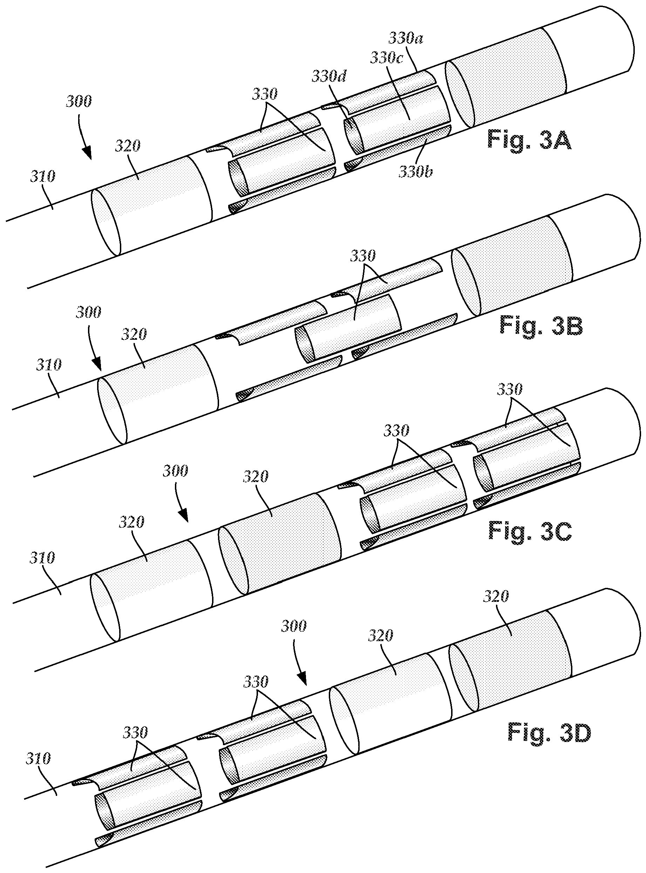

When the lead 100 includes both ring electrodes 120 and segmented electrodes 130, the ring electrodes 120 and the segmented electrodes 130 may be arranged in any suitable configuration. For example, when the lead 100 includes two sets of ring electrodes 120 and two sets of segmented electrodes 130, the ring electrodes 120 can flank the two sets of segmented electrodes 130 (see *e.g*., Figure 1). Alternately, the two sets of ring electrodes 120 can be disposed proximal to the two sets of segmented electrodes 130 (see *e.g.*, Figure 3C), or the two sets of ring electrodes 120 can be disposed distal to the two sets of segmented electrodes 130 (see *e.g*., Figure 3D). One of the ring electrodes can be a tip electrode (see, tip electrode 320a of Figures 3E and 3G). It will be understood that other configurations are possible as well (*e.g*., alternating ring and segmented electrodes, or the like).

By varying the location of the segmented electrodes 130, different coverage of the target neurons may be selected. For example, the electrode arrangement of Figure 3C may be useful if the physician anticipates that the neural target will be closer to a distal tip of the lead body 110, while the electrode arrangement of Figure 3D may be useful if the physician anticipates that the neural target will be closer to a proximal end of the lead body 110.

Any combination of ring electrodes 120 and segmented electrodes 130 may be disposed on the lead 100. For example, the lead may include a first ring electrode 120, two sets of segmented electrodes; each set formed of four segmented electrodes 130, and a final ring electrode 120 at the end of the lead. This configuration may simply be referred to as a 1-4-4-1 configuration (Figures 3A and 3E). It may be useful to refer to the electrodes with this shorthand notation. Thus, the embodiment of Figure 3C may be referred to as a 1-1-4-4 configuration, while the embodiment of Figure 3D may be referred to as a 4-4-1-1 configuration. The embodiments of Figures 3F and 3G can be referred to as a 1-3-3-1 configuration. Other electrode configurations include, for example, a 2-2-2-2 configuration, where four sets of segmented electrodes are disposed on the lead, and a 4-4 configuration, where two sets of segmented electrodes, each having four segmented electrodes 130 are disposed on the lead. The 1-3-3-1 electrode configuration of Figures 3F and 3G has two sets of segmented electrodes, each set containing three electrodes disposed around the circumference of the lead, flanked by two ring electrodes (Figure 3F) or a ring electrode and a tip electrode (Figure 3G). In some embodiments, the lead includes 16 electrodes. Possible configurations for a 16-electrode lead include, but are not limited to 4-4-4-4; 8-8; 3-3-3-3-3-1 (and all rearrangements of this configuration); and 2-2-2-2-2-2-2-2.

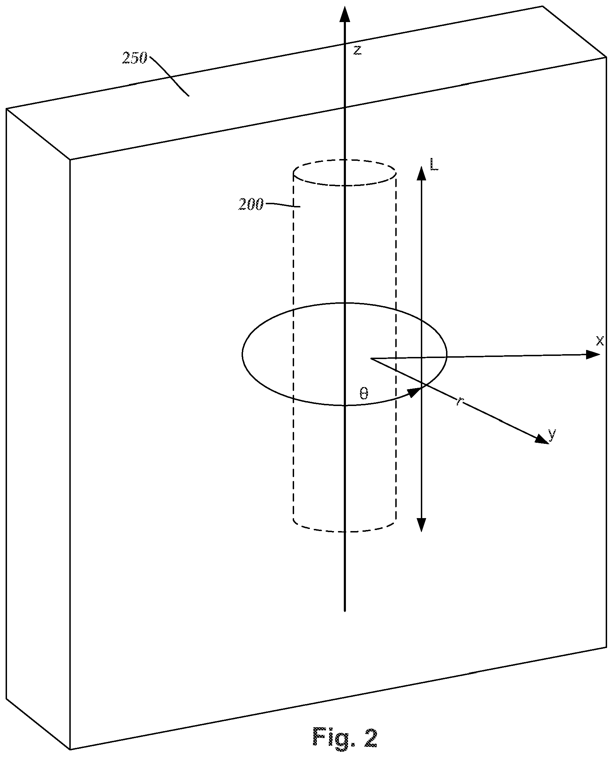

Figure 2 is a schematic diagram to illustrate radial current steering along various electrode levels along the length of the lead 200. While conventional lead configurations with ring electrodes are only able to steer current along the length of the lead (the *z*-axis), the segmented electrode configuration is capable of steering current in the *x*-axis, *y*-axis as well as the *z*-axis. Thus, the centroid of stimulation may be steered in any direction in the three-dimensional space surrounding the lead 200. In some embodiments, the radial distance, *r*, and the angle θ around the circumference of the lead 200 may be dictated by the percentage of anodic current (recognizing that stimulation predominantly occurs near the cathode, although strong anodes may cause stimulation as well) introduced to each electrode. In at least some embodiments, the configuration of anodes and cathodes along the segmented electrodes allows the centroid of stimulation to be shifted to a variety of different locations along the lead 200.

As can be appreciated from Figure 2, the centroid of stimulation can be shifted at each level along the length of the lead 200. The use of multiple sets of segmented electrodes at different levels along the length of the lead allows for three-dimensional current steering. In some embodiments, the sets of segmented electrodes are shifted collectively (*i.e*., the centroid of simulation is similar at each level along the length of the lead). In at least some other embodiments, each set of segmented electrodes is controlled independently. Each set of segmented electrodes may contain two, three, four, five, six, seven, eight or more segmented electrodes. It will be understood that different stimulation profiles may be produced by varying the number of segmented electrodes at each level. For example, when each set of segmented electrodes includes only two segmented electrodes, uniformly distributed gaps (inability to stimulate selectively) may be formed in the stimulation profile. In some embodiments, at least three segmented electrodes 230 in a set are utilized to allow for true 360° selectivity.

As previously indicated, the foregoing configurations may also be used while utilizing recording electrodes. In some embodiments, measurement devices coupled to the muscles or other tissues stimulated by the target neurons or a unit responsive to the patient or clinician can be coupled to the control unit or microdrive motor system. The measurement device, user, or clinician can indicate a response by the target muscles or other tissues to the stimulation or recording electrodes to further identify the target neurons and facilitate positioning of the stimulation electrodes. For example, if the target neurons are directed to a muscle experiencing tremors, a measurement device can be used to observe the muscle and indicate changes in tremor frequency or amplitude in response to stimulation of neurons. Alternatively, the patient or clinician may observe the muscle and provide feedback.

The reliability and durability of the lead will depend heavily on the design and method of manufacture. Fabrication techniques discussed below provide methods that can produce manufacturable and reliable leads.

Returning to Figure 1, when the lead 100 includes a plurality of sets of segmented electrodes 130, it may be desirable to form the lead 100 such that corresponding electrodes of different sets of segmented electrodes 130 are radially aligned with one another along the length of the lead 100 (see *e.g.,* the segmented electrodes 130 shown in Figure 1). Radial alignment between corresponding electrodes of different sets of segmented electrodes 130 along the length of the lead 100 may reduce uncertainty as to the location or orientation between corresponding segmented electrodes of different sets of segmented electrodes. Accordingly, it may be beneficial to form electrode arrays such that corresponding electrodes of different sets of segmented electrodes along the length of the lead 100 are radially aligned with one another and do not radially shift in relation to one another during manufacturing of the lead 100.

In other embodiments, individual electrodes in the two sets of segmented electrodes 130 are staggered (see, Figure 3B) relative to one another along the length of the lead body 110. In some cases, the staggered positioning of corresponding electrodes of different sets of segmented electrodes along the length of the lead 100 may be designed for a specific application.

Segmented electrodes can be used to tailor the stimulation region so that, instead of stimulating tissue around the circumference of the lead as would be achieved using a ring electrode, the stimulation region can be directionally targeted. In some instances, it is desirable to target a parallelepiped (or slab) region 250 that contains the electrodes of the lead 200, as illustrated in Figure 2. One arrangement for directing a stimulation field into a parallelepiped region uses segmented electrodes disposed on opposite sides of a lead.

Figures 3A-3E illustrate leads 300 with segmented electrodes 330, optional ring electrodes 320 or tip electrodes 320a, and a lead body 310. The sets of segmented electrodes 330 include either two (Figure 3B) or four (Figures 3A, 3C, and 3D) or any other number of segmented electrodes including, for example, three, five, six, or more.

Any other suitable arrangements of segmented electrodes can be used. As an example, arrangements in which segmented electrodes are arranged helically with respect to each other. At least some embodiments include a double helix.

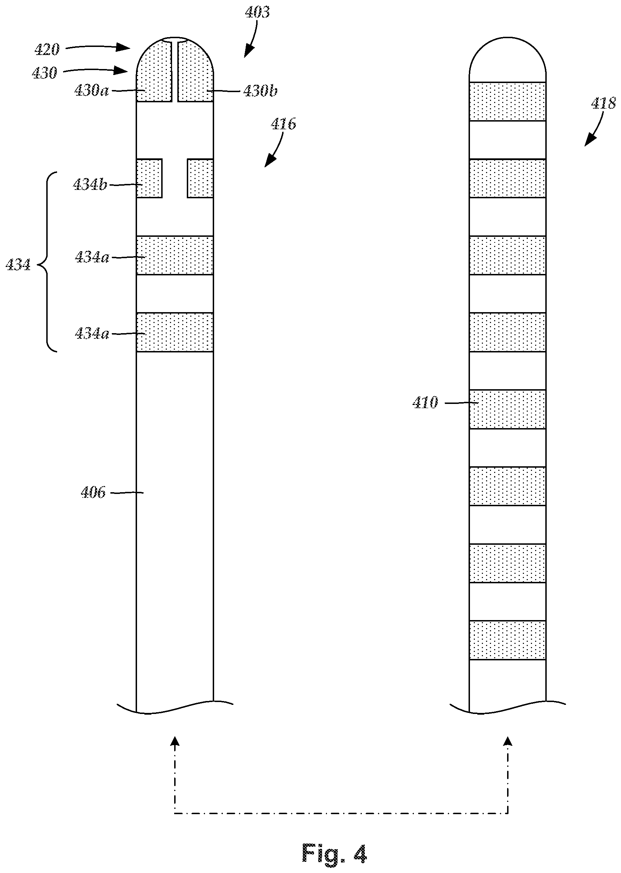

Turning to Figure 4, one challenge in making leads with tip electrodes is to provide current steering around a distal tip of a lead. As herein described, a set of segmented tip electrodes can be disposed along a distal tip of a lead. The set of segmented tip electrodes enable radial steering of current around the tip of the lead. In at least some embodiments, the set of segmented tip electrodes can be formed from a pre-tip-electrode that includes segmented tip electrodes attached to a base ring. In at least some embodiments, the pre-tip-electrode can be disposed along the lead by forming the lead body around the segmented tip electrodes. After forming the lead body, the base ring can be removed to separate the segmented tip electrodes.

Figure 4 illustrates a side view of one embodiment of a distal end portion 416 and a proximal end portion 418 of a lead body 406 of a lead 403. The distal end portion 416 of the lead body 406 includes a distal tip 420. Terminals, such as terminal 410, are disposed along the proximal end portion 418 of the lead body 406.

A set of segmented tip electrodes 430 is disposed along the distal tip 420 of the lead body 406. In Figure 4, the set of segmented tip electrodes 430 is shown having two individual segmented tip electrodes 430a and 430b. The individual segmented tip electrodes of the set of tip segmented electrodes 430 are each disposed around a different portion of the circumference of the distal tip 420 of the lead body 406 and are physically and electrically isolated from one another. The set of segmented tip electrodes 430 can include any suitable number of individual segmented tip electrodes including, for example, two, three, four, five, six, or more individual segmented tip electrodes. In at least some embodiments, the set of segmented tip electrodes 430 has a rounded distal end. It may be advantageous to form the set of segmented tip electrodes with a rounded distal end to facilitate implantation and potentially reduce patient discomfort during operation caused by the distal end contacting patient tissue.

One or more non-tip electrodes (*i.e*., electrodes disposed along a portion of the lead body other than the distal tip) may also be disposed along the lead. Figure 4 shows non-tip electrodes 434 disposed along the distal end portion 416 of the lead body 406 proximal to the distal tip 420. In Figure 4, the non-tip electrodes 434 include multiple ring electrodes 434a and multiple sets of non-tip segmented electrodes 434b. Any suitable number of non-tip electrodes 434 can be disposed along the distal end portion 416 of the lead including, for example, one, two, three, four, five, six, seven, eight, nine, ten, eleven, twelve, fourteen, sixteen, twenty, twenty-four, or more non-tip electrodes 434. The total number of non-tip electrodes 434 can include any combination of ring electrodes and segmented electrodes, including all ring electrodes and no segmented electrodes, or all segmented electrodes and no ring electrodes.

As with the tip segmented electrodes, the individual non-tip electrodes of the set of non-tip segmented electrodes 434b are each disposed around a particular circumference of the lead body 406 and are physically and electrically isolated from one another. The set of non-tip segmented electrodes 434b can include any suitable number of individual segmented electrodes including, for example, two, three, four, five, six, or more individual segmented electrodes. In at least some embodiments, a single segmented electrode is disposed around a portion of a particular circumference of the lead body that is not part of a set of segmented electrodes.

In at least some embodiments, the non-tip electrodes 434 are isodiametric with the lead body 406. In at least some embodiments, the set of tip electrodes 430 is isodiametric with the lead body 406. In at least some embodiments, the non-tip electrodes 434 and the set of tip electrodes 430 are each isodiametric with the lead body 406.

The non-tip electrodes 434 can be disposed along the distal end portion 416 of the lead body 406 in any suitable configuration. In at least some embodiments, the distal-most non-tip electrode 434 is a set of non-tip segmented electrodes 434b. In at least some other embodiments, the distal-most non-tip electrode 334 is a ring electrode 334a.

In at least some embodiments, the lead body 406 is formed by molding the lead body 406 between the non-tip electrodes 434 and, at least in some embodiments, between the non-tip electrodes 434 and the terminals 410. The material of the lead body 406 can also be molded between the distal-most non-tip electrode 434 and the set of tip segmented electrodes 430.

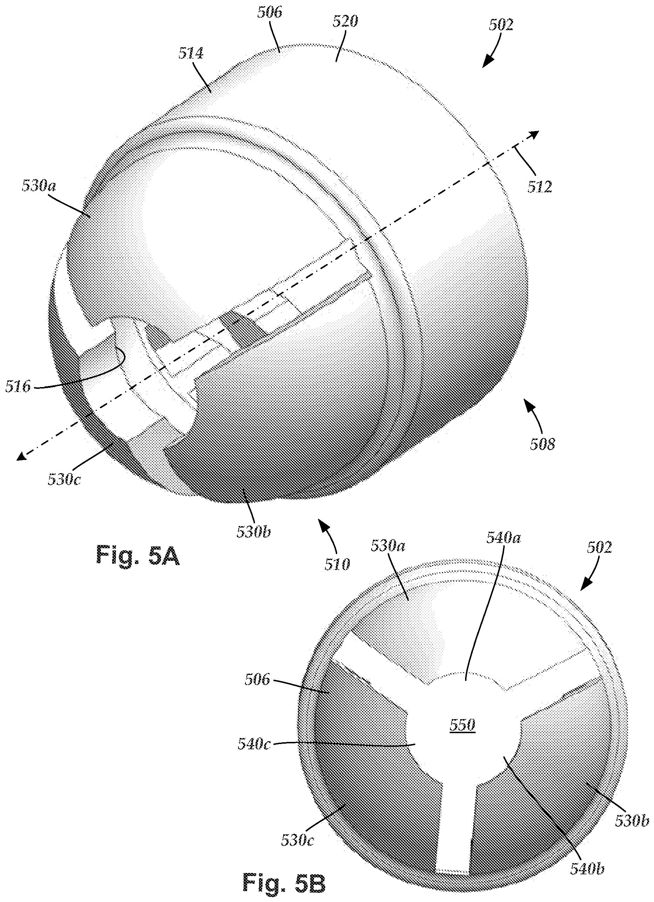

During the molding process, the material that will form the lead body can flow into an injection aperture (550 in Figures 5A-6B) defined along the set of tip electrodes, or along a pre-tip-electrode (502 in Figure 5A) used to form the set of tip electrodes, or both. Any molding process can be used including, but not limited to, injection molding, or epoxy filling, or the like or combinations thereof. The lead body 406 can be formed of any material that can be molded by flowing the material around the other components and then solidify the material to form the lead body. Any suitable process can be used to solidify the material including, but not limited to, cooling the material, photo-curing, heat curing, cross-linking, and the like. Examples of suitable materials can include silicone, polyurethane, polyetheretherketone, epoxy, and the like.

Figure 5A schematically illustrates, in perspective view, one embodiment of a pre-tip-electrode 502. Figure 5B schematically illustrates, in end view, one embodiment of the pre-tip-electrode 502. The pre-tip-electrode 502 has a pre-tip-electrode body 506 with a proximal end 508, a distal end portion 510, a longitudinal axis 512, a circumference, an outer surface 514, and an inner surface 516 opposite to the outer surface 514. In at least some embodiments, the pre-tip-electrode body 506 is dome-shaped, or substantially dome-shaped.

The pre-tip-electrode body 506 includes a base portion 520 and multiple segmented tip electrodes 530a, 530b, and 530c coupled to the base portion 520. The base portion 520 is ring-shaped, or substantially ring-shaped. In at least some embodiments, the base portion 520 is close-looped (*i.e.,* the base portion 520 extends around an entire circumference). In other embodiments, the base portion 520 is open-looped (*i.e.,* the base portion 520 extends around less than an entire circumference). In at least some embodiments, the base portion 520 is disposed at the proximal end 508 of the pre-tip-electrode body 506.

The segmented tip electrodes 530a, 530b, and 530c are circumferentially spaced-apart from one another and are attached to the base portion 520 such that the segmented tip electrodes 530a, 530b, and 530c extend distally from the base portion 530. In at least some embodiments, the segmented tip electrodes 530a, 530b, and 530c collectively form a hemispherical, or substantially hemispherical, structure. In at least some embodiments, the segmented tip electrodes 530a, 530b, and 530c are configured and arranged to maintain a constant positioning relative to each other during operation. In a least some embodiments, the segmented tip electrodes 530a, 530b, and 530c are physically, or electrically, or both, coupled to one another solely via the base portion 520.

In at least some embodiments, the segmented tip electrodes 530a, 530b, and 530c are curved inwardly as they extend distally along the longitudinal axis 512 from the base portion 520 such that each of the segmented tip electrodes 530a, 530b, and 530c has an arc-shaped longitudinal cross-sectional profile. In at least some embodiments, the segmented tip electrodes 530a, 530b, and 530c are each curved about (*e.g*., transverse to) the longitudinal axis 512 such that each of the segmented tip electrodes 530a, 530b, and 530c has an arc-shaped transverse cross-sectional profile. In at least some embodiments, the segmented tip electrodes 530a, 530b, and 530c are each curved along the longitudinal axis 512 and also along a transverse axis transverse to the longitudinal axis 512.

Optionally, one or more cutouts 540a, 540b, and 540c are defined along one or more of the segmented tip electrodes 530a, 530b, and 530c, respectively, to form an injection aperture 550. In Figures 5A-5B, a cutout is defined along each of the segmented tip electrodes 530a, 530b, and 530c. Figures 5A-5B also show the injection aperture 550 defined along distal-most portions of the segmented tip electrodes 530a, 530b, and 530c. It will be understood that the cutouts 540a, 540b, and 540c can be formed along any portion of any number of the segmented tip electrodes 530a, 530b, and 530c.

The pre-tip-electrode body may be made using a metal, alloy, conductive oxide, or any other suitable conductive biocompatible material. Examples of suitable materials include, but are not limited to, platinum, platinum-iridium alloy, iridium, titanium, tungsten, palladium, palladium-rhodium, 616L stainless steel (or any other suitable stainless steel), tantalum, Nitinol, iridium-rhodium, or a conductive polymer or the like. Preferably, the pre-tip-electrode body is made of a material that is biocompatible and does not substantially corrode under expected operating conditions in the operating environment for the expected duration of use. The pre-tip-electrode body can be formed using any suitable method including, for example, machining, electrical discharge machining, stamping, etching, laser cutting, or the like or combinations thereof.

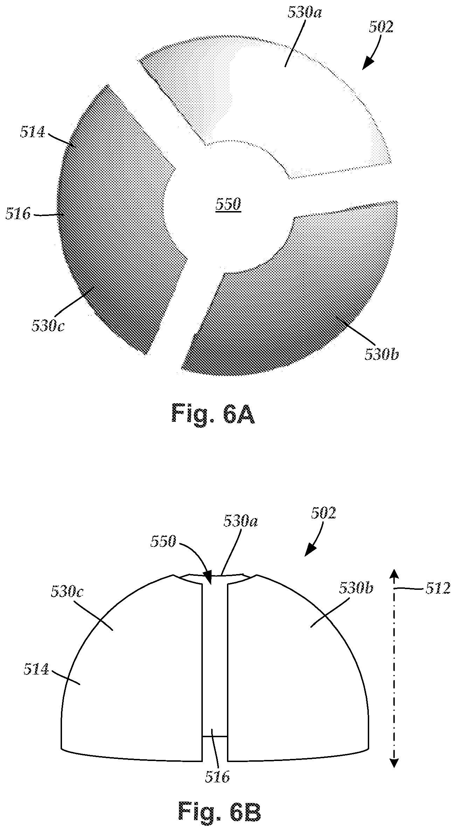

Turning to Figures 6A-6B, the base portion of the pre-tip-electrode body can be removed (for example, by grinding, machining, etching, ablating, or otherwise removing the base portion) to leave the separated segmented tip electrodes when the pre-tip-electrode is in place on the lead. Figure 6A illustrates, in end view, one embodiment of the segmented tip electrodes 530a, 530b, and 530c after removal of the base portion 520. Figure 6B illustrates, in side view, one embodiment of the segmented tip electrodes 530a, 530b, and 530c after removal of the base portion 520.

One or more tip-electrode conductors (not shown) are attached, welded, soldered, or otherwise electrically coupled to each of the segmented tip electrodes. In at least some embodiments, the one or more tip-electrode conductors are coupled to the inner surfaces 516 of segmented tip electrodes. The coupling of the tip-electrode conductors to the segmented tip electrodes may occur either before or after formation of the lead body 306. The tip-electrode conductor, like other conductors in the lead, extends along the lead and is electrically coupled to one of the terminals disposed along the proximal end portion of the lead.

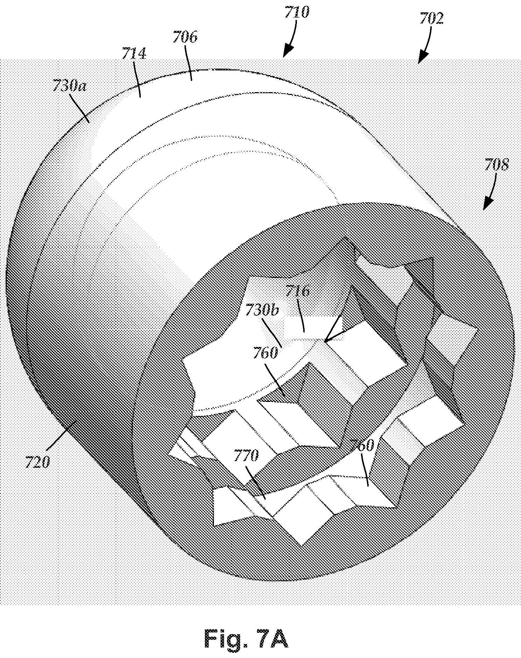

Turning to Figures 7A-7B, in at least some embodiments the pre-tip electrode body includes one or more lead-retention features, such as one or more channels, longitudinal grooves, or the like that are defined along the inner surfaces of the base portion, the segmented tip electrodes, or both, and that facilitate maintenance of the positioning and spacing, as well as retention, of the pre-tip electrode body, or the segmented tip electrodes, or both.

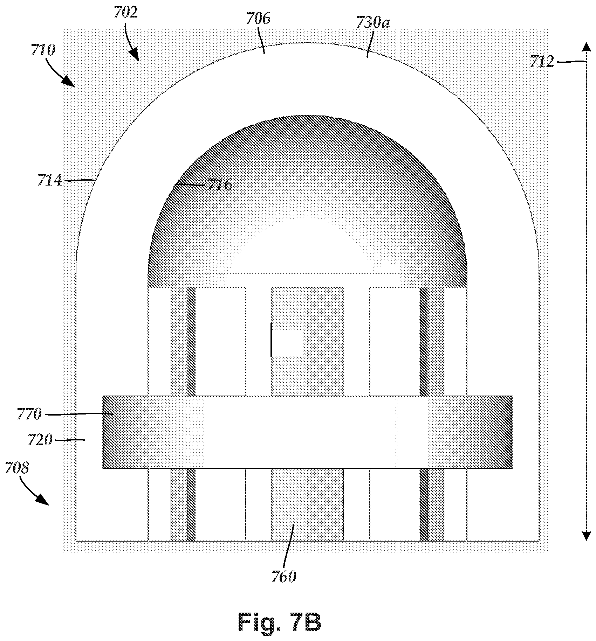

In at least some embodiments, the lead-retention features include one or more grooves, such as one or more longitudinal grooves, a transverse channel, or both. Figure 7A illustrates a schematic perspective view of one embodiment of a pre-tip electrode 702. Figure 7B illustrates a schematic longitudinal cross-section of the pre-tip-electrode 702. The pre-tip-electrode 702 includes a pre-tip-electrode body 706 having a proximal end 708, a distal end 710, a longitudinal axis 712, a circumference, an outer surface 714, and an inner surface 716 opposite to the outer surface 714. The pre-tip-electrode body 706 includes a base portion 720 and segmented tip electrodes 730a and 730b coupled to the base portion 720.

As mentioned above, when the lead body is formed, the material of the lead body flows into the pre-tip-electrode body and solidifies. The one or more lead-retention features form shapes that, when material of the lead body is flowed into and solidifies, are configured and arranged to facilitate retention of the pre-tip-electrode body (and the set of segmented tip electrodes formed therefrom) on the lead body.

In at least some embodiments, the pre-tip electrode 702 defines multiple longitudinal grooves, such as longitudinal groove 760. The longitudinal grooves 760 are defined along the inner surface 716 of the pre-tip-electrode body 706 and extend deeper into the pre-tip-electrode body 706 than adjacent portions of the inner surface 716. The longitudinal grooves 760 are configured and arranged to facilitate retention of the pre-tip electrode 702 on the lead body 406 and also, in at least some embodiments, to resist rotation of the pre-tip electrode 702 around the lead body 406.

Any suitable number of longitudinal grooves can be defined along the inner surface 716 of the pre-tip-electrode body 706 including, for example, two, three, four, five, six, seven, eight, nine, ten, eleven, twelve, thirteen, fourteen, fifteen, sixteen, seventeen, eighteen, nineteen, twenty, or more longitudinal grooves 760. The longitudinal grooves can be defined along any suitable locations of the inner surface 716 of the pre-tip-electrode body 706. In at least some embodiments, at least one of the longitudinal grooves is defined at least partially along the segmented-tip-electrode portions of the pre-tip-electrode body. In at least some embodiments, at least one of the longitudinal grooves is defined at least partially along the base portion of the pre-tip-electrode body.

The longitudinal grooves 760 can be any suitable shape. In at least some embodiments, the longitudinal grooves are elongated such that the longitudinal grooves have lengths that are at least 2, 3, 4, 5, 10, 15, 20, or more times widths of the longitudinal grooves. In at least some embodiments, the longitudinal grooves each extend parallel to one another. In at least some embodiments, at least one of the longitudinal grooves extends in a different direction than at least one of the other longitudinal grooves. In at least some embodiments, the longitudinal grooves each extend in a direction that is parallel, or substantially parallel, to the longitudinal axis 712 of the pre-tip-electrode body 706.

The longitudinal grooves can be of any suitable length. In some embodiments, the longitudinal grooves extend an entire length of the pre-tip-electrode body 706. In other embodiments, the longitudinal grooves extend less than an entire length of the pre-tip-electrode body 706. In at least some embodiments, the longitudinal grooves extend at least 25%, 30%, 35%, 40%, 45%, 50%, 60%, or more, of a longitudinal length of the pre-tip-electrode body 706.

In some embodiments, at least one of the longitudinal grooves extends partially through a thickness of the pre-tip-electrode body 706 and does not open to the outer surface 714. In at least some embodiments, at least one of the longitudinal grooves extends entirely through the thickness of the pre-tip-electrode body 706 and opens to the outer surface 714. In Figures 7A-7B, each of the longitudinal grooves 760 is shown extending partially through the thickness of the pre-tip-electrode body 706 from the inner surface 716 of the pre-tip-electrode body 706 and does not open to the outer surface 714.

When material of the lead body flows into the pre-tip-electrode body 706, some of the material flows from the inner surface 716 of the pre-tip-electrode body 706 into the longitudinal grooves 760. Once the material solidifies, the pre-tip electrode 702 is prevented from rotating and from being removed from the lead body 406. When the base portion 720 is ground down to separate the segmented tip electrodes, the segmented tip electrodes are, likewise, prevented from rotating and from being removed from the lead body 406.

In at least some embodiments, the tip electrode defines one or more channels 770 extending along the inner surface 716 of the pre-tip-electrode body 706. The one or more channels 770 extend deeper into the pre-tip-electrode body 706 than adjacent portions of the inner surface 716. The one or more channels 770 are configured and arranged to facilitate retention of the pre-tip-electrode along the lead body. When material of the lead body flows into the pre-tip-electrode body 706, some of the material flowing into the pre-tip-electrode body 706 flows into the one or more channels 770. Once the material solidifies, the material resists movement of the pre-tip electrode relative to the lead body.

Any suitable number of channels 770 can be defined along the inner surface 716 of the pre-tip-electrode body 706 including, for example, one, two, three, four, five, or more channels 770. The channels 770 can be defined along any suitable portions of the inner surface 716 of the pre-tip-electrode body 706. In at least some embodiments, at least one of the channels is defined at least partially along the segmented-tip-electrode portions of the pre-tip-electrode body. In at least some embodiments, at least one of the channels is defined at least partially along the base portion of the pre-tip-electrode body. In at least some embodiments, at least one of the one or more channels 770 extends through at least a portion of the inner surface 716 of the pre-tip-electrode body 706 along which at least one of the longitudinal grooves 760 also extends. In other words, at least one of the one or more channels 770 intersects at least one of the longitudinal grooves 760.

In at least some embodiments, the one or more channels 770 extend along at least a portion of the circumference of the pre-tip-electrode body 706. In at least some embodiments, at least one of the one or more channels 770 extends at least 25%, 50%, or 75% around the circumference of the pre-tip-electrode. In at least some embodiments, at least one of the one or more channels 770 extends around the entire circumference of the pre-tip-electrode. In at least some embodiments, at least one of the channels 770 is defined along the proximal end portion of the pre-tip-electrode body 706. In Figures 7A-7B, a single channel 770 is shown extending around the entire circumference of the tip electrode along the proximal end portion of the pre-tip-electrode body 706.

In at least some embodiments, the one or more channels 770 extend more deeply into the inner surface 716 of the pre-tip-electrode body 706 than at least one of the longitudinal grooves 760. In which case, for example, when one of the one or more of the channels 770 intersects a particular longitudinal groove, and when that channel extends more deeply into the inner surface 716 of the pre-tip-electrode body 706 than the longitudinal groove, that channel separates that longitudinal groove into a proximal portion and a distal portion. In Figures 7A-7B, the single channel 770 is shown extending around the entire circumference of the pre-tip-electrode body 706 and extending deeper into the inner surface 716 of the pre-tip-electrode body 706 than the longitudinal grooves 760 such that each of the longitudinal grooves 760 is separated into a proximal portion and a distal portion.

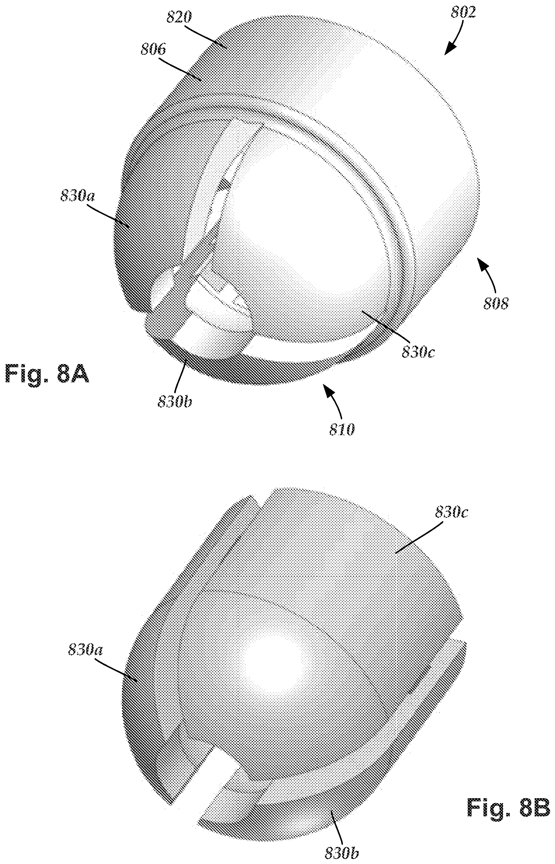

Turning to Figures 8A-8D, in alternate embodiments the base portion of a pre-tip-electrode body is disposed over a proximal end portion of the segmented-tip-electrode portions of the pre-tip-electrode body. Removal of the base portion (for example, by grinding, machining, etching, ablating, or otherwise removing the base portion) separates the segmented tip electrodes physically and electrically from one another, as explained above. When the segmented-tip-electrode portions of the pre-tip-electrode body extend beneath the base portion in addition to extending distally from the base portion, removal of the base portion results in the formed segmented tip electrodes having a larger longitudinal length than the segmented tip electrodes 530a-c.

Figure 8A schematically illustrates, in distal perspective view, another embodiment of a pre-tip-electrode 802. The pre-tip-electrode 802 has a pre-tip-electrode body 806 with a proximal end portion 808 and a distal end portion 810. The pre-tip-electrode body 806 includes a base portion 820 and multiple segmented tip electrodes 830a, 830b, and 830c coupled to the base portion 820.

The segmented tip electrodes 830a, 830b, and 830c are circumferentially spaced-apart from one another and are attached to the base portion 820 such that the segmented tip electrodes 830a, 830b, and 830c extend distally from the base portion 530 and also beneath a portion of the base portion 820. In some embodiments, the segmented tip electrodes 830a, 830b, and 830c extend beneath the base portion 820 such that the segmented tip electrodes 830a, 830b, and 830c extend to a proximal end of the base portion 820. In a least some embodiments, the segmented tip electrodes 830a, 830b, and 830c are physically, or electrically, or both, coupled to one another solely via the base portion 820.

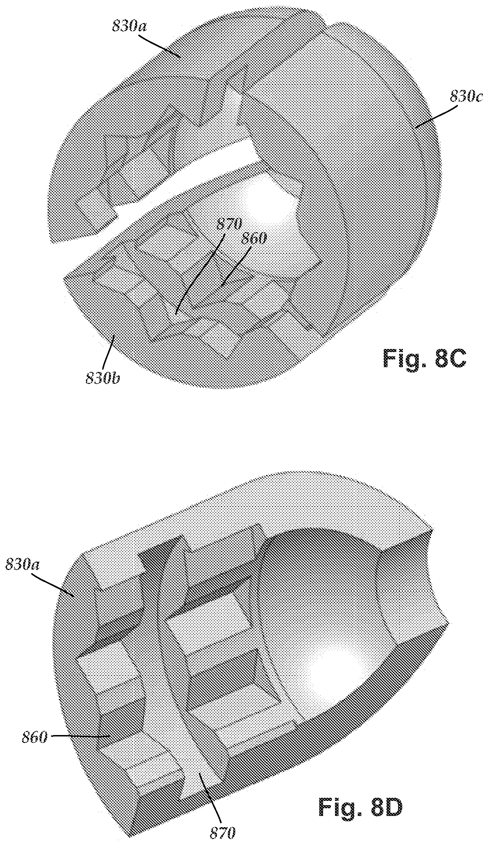

Figure 8B schematically illustrates, in distal perspective view, one embodiment of the segmented tip electrodes 830a, 830b, and 830c after removal of the base portion 820. Figure 8C schematically illustrates, in proximal perspective view, one embodiment of the segmented tip electrodes 830a, 830b, and 830c after removal of the base portion 820. Figure 8D schematically illustrates, in perspective side view, one embodiment of the segmented tip electrode 830a. In at least some embodiments, the segmented tip electrodes include one or more lead-retention features, such as one or more transverse channels 870, or one or more longitudinal grooves 860, or both, that are defined along inner surfaces of the segmented tip electrodes 830a, 830b, and 830c and that facilitate maintenance of the positioning and spacing, as well as retention, of the segmented tip electrodes 830a, 830b, and 830c along a lead body.

The above specification, examples and data provide a description of the manufacture and use of the composition of the invention. Since many embodiments of the invention can be made without departing from the scope of the invention, the invention also resides in the claims hereinafter appended.

## Claims

1. An implantable electrical stimulation lead (403), comprising:
a lead body (406) having a proximal end portion, a distal end portion, a distal tip, a longitudinal length, and a longitudinal surface;
a set of segmented tip electrodes (430; 530a, 530b, 530c, 730a, 730b; 830a, 830b, 830c) that are disposed circumferentially about the distal tip of the lead body and that are electrically-isolated from each other, each segmented tip electrode of the set of segmented tip electrodes having an inner surface, an opposing outer stimulating surface exposed along the longitudinal surface of the lead body, and at least one channel defined in the inner surface and extending along at least a portion of a circumference of the segmented tip electrodes, wherein a portion of the lead body is disposed against the inner surfaces of each of the segmented tip electrodes and circumferentially between each of the segmented tip electrodes;
at least one non-tip electrode disposed along the distal end portion of the lead body proximal to the set of segmented tip electrodes;
a plurality of terminals disposed along the proximal end portion of the lead body; and
a plurality of conductors, each conductor of the plurality of conductors electrically coupling the plurality of terminals to each of the segmented tip electrodes and the at least one non-tip electrode.

2. The implantable electrical stimulation lead of claim 1, wherein each segmented tip electrode of the set of segmented tip electrodes has an arc-shaped cross-section along an axis parallel to the longitudinal length of the lead body at the distal tip.

3. The implantable electrical stimulation lead of claim 1, wherein each segmented tip electrode of the set of segmented tip electrodes has an arc-shaped cross-section along an axis transverse to the longitudinal length of the lead body at the distal tip.

4. The implantable electrical stimulation lead of claim 1, wherein each segmented tip electrode of the set of segmented tip electrodes has an arc-shaped cross-section along each of an axis parallel to the longitudinal length of the lead body at the distal tip and an axis transverse to the longitudinal length of the lead body at the distal tip.

5. The implantable electrical stimulation lead of claim 1, wherein the outer stimulating surface of each segmented tip electrode of the set of segmented tip electrodes is flush with the longitudinal surface of the lead body.

6. The implantable electrical stimulation lead of claim 1, further comprising a lead-retention feature formed along the inner surface of at least one segmented tip electrode of the set of segmented tip electrodes, the lead-retention feature extending deeper into the inner surface of the at least one segmented tip electrode than adjacent portions of the inner surface.

7. The implantable electrical stimulation lead of claim 6, wherein the lead-retention feature comprises a plurality of grooves extending along the inner surface of the at least one segmented tip electrode along a longitudinal axis parallel to the longitudinal length of the lead body at the distal tip.

8. The implantable electrical stimulation lead of claim 7, wherein the at least one channel intersects at least a portion of at least one of the plurality of grooves.

9. The implantable electrical stimulation lead of claim 1, wherein the plurality of non-tip electrodes comprises at least one set of non-tip segmented electrodes or at least one ring electrode.

10. An electrical stimulation system comprising:
the implantable electrical stimulation lead of claim 1;
a control module coupleable to the implantable electrical stimulation lead, the control module comprising
a housing, and
an electronic subassembly disposed in the housing; and
a connector for receiving the implantable electrical stimulation lead, the connector comprising
a connector housing defining a port, the port configured and arranged for receiving the proximal end portion of the lead body of the implantable electrical stimulation lead, and
a plurality of connector contacts disposed in the port, the plurality of connector contacts configured and arranged to couple to the plurality of terminals disposed along the proximal end portion of the lead body when the proximal end portion of the lead body is received by the port.

11. The electrical stimulation system of claim 10, further comprising a lead extension coupling the implantable electrical stimulation lead to the control module.

## Patentansprüche

1. Implantierbare elektrische Stimulationsleitung (403), die aufweist:
einen Leitungskörper (406) mit einem proximalen Endabschnitt, einem distalen Endabschnitt, einer distalen Spitze, einer Längslänge und einer Längsfläche;
einen Satz segmentierter Spitzenelektroden (430; 530a, 530b, 530c, 730a, 730b; 830a, 830b, 830c), die um die distale Spitze des Leitungskörpers über den Umfang angeordnet sind und die voneinander elektrisch isoliert sind, wobei jede segmentierte Spitzenelektrode des Satzes segmentierter Spitzenelektroden eine Innenfläche, eine gegenüberliegende Außenstimulationsfläche, die entlang der Längsfläche des Leitungskörpers freiliegt, und mindestens einen Kanal hat, der in der Innenfläche gebildet ist und sich entlang mindestens eines Abschnitts eines Umfangs der segmentierten Spitzenelektroden erstreckt, wobei ein Abschnitt des Leitungskörpers an den Innenflächen jeder der segmentierten Spitzenelektroden und über den Umfang zwischen jeder der segmentierten Spitzenelektroden angeordnet ist;
mindestens eine Nichtspitzenelektrode, die entlang des distalen Endabschnitts des Leitungskörpers proximal zum Satz segmentierter Spitzenelektroden angeordnet ist;
mehrere Anschlüsse, die entlang des proximalen Endabschnitts des Leitungskörpers angeordnet sind; und
mehrere Leiter, wobei jeder Leiter der mehreren Leiter die mehreren Anschlüsse mit jeder der segmentierten Spitzenelektroden und der mindestens einen Nichtspitzenelektrode elektrisch koppelt.

2. Implantierbare elektrische Stimulationsleitung nach Anspruch 1, wobei jede segmentierte Spitzenelektrode des Satzes segmentierter Spitzenelektroden einen bogenförmigen Querschnitt entlang einer Achse parallel zur Längslänge des Leitungskörpers an der distalen Spitze hat.

3. Implantierbare elektrische Stimulationsleitung nach Anspruch 1, wobei jede segmentierte Spitzenelektrode des Satzes segmentierter Spitzenelektroden einen bogenförmigen Querschnitt entlang einer Achse quer zur Längslänge des Leitungskörpers an der distalen Spitze hat.

4. Implantierbare elektrische Stimulationsleitung nach Anspruch 1, wobei jede segmentierte Spitzenelektrode des Satzes segmentierter Spitzenelektroden einen bogenförmigen Querschnitt entlang jeweils einer Achse parallel zur Längslänge des Leitungskörpers an der distalen Spitze und einer Achse quer zur Längslänge des Leitungskörpers an der distalen Spitze hat.

5. Implantierbare elektrische Stimulationsleitung nach Anspruch 1, wobei die Außenstimulationsfläche jeder segmentierten Spitzenelektrode des Satzes segmentierter Spitzenelektroden bündig mit der Längsfläche des Leitungskörpers ist.

6. Implantierbare elektrische Stimulationsleitung nach Anspruch 1, ferner mit einem Leitungshaltemerkmal, das entlang der Innenfläche mindestens einer segmentierten Spitzenelektrode des Satzes segmentierter Spitzenelektroden gebildet ist, wobei sich das Leitungshaltemerkmal tiefer in die Innenfläche der mindestens einen segmentierten Spitzenelektrode als benachbarte Abschnitte der Innenfläche erstreckt.

7. Implantierbare elektrische Stimulationsleitung nach Anspruch 6, wobei das Leitungshaltemerkmal mehrere Nuten aufweist, die sich entlang der Innenfläche der mindestens einen segmentierten Spitzenelektrode entlang einer Längsachse parallel zur Längslänge des Leitungskörpers an der distalen Spitze erstrecken.

8. Implantierbare elektrische Stimulationsleitung nach Anspruch 7, wobei der mindestens eine Kanal mindestens einen Abschnitt mindestens einer der mehreren Nuten schneidet.

9. Implantierbare elektrische Stimulationsleitung nach Anspruch 1, wobei die mehreren Nichtspitzenelektroden mindestens einen Satz segmentierter Nichtspitzenelektroden oder mindestens eine Ringelektrode aufweisen.

10. Elektrisches Stimulationssystem, das aufweist:
die implantierbare elektrische Stimulationsleitung nach Anspruch 1;
ein Steuermodul, das mit der implantierbaren elektrischen Stimulationsleitung koppelbar ist, wobei das Steuermodul aufweist:
ein Gehäuse und
eine elektronische Teilanordnung, die im Gehäuse angeordnet ist; und einen Verbinder zum Aufnehmen der implantierbaren elektrischen Stimulationsleitung, wobei der Verbinder aufweist:
ein Verbindergehäuse, das einen Port bildet, wobei der Port zum Aufnehmen des proximalen Endabschnitts des Leitungskörpers der implantierbaren elektrischen Stimulationsleitung konfiguriert und angeordnet ist, und
mehrere Verbinderkontakte, die im Port angeordnet sind, wobei die mehreren Verbinderkontakte so konfiguriert und angeordnet sind, dass sie sich mit den mehreren Anschlüssen koppeln, die entlang des proximalen Endabschnitts des Leitungskörpers angeordnet sind, wenn der proximale Endabschnitt des Leitungskörpers durch den Port aufgenommen ist.

11. Elektrisches Stimulationssystem nach Anspruch 10, ferner mit einer Leitungsverlängerung, die die implantierbare elektrische Stimulationsleitung mit dem Steuermodul koppelt.

## Revendications

1. Fil de stimulation électrique implantable (403) comprenant :
un corps de fil (406) ayant une partie d'extrémité proximale, une partie d'extrémité distale, une pointe distale, une longueur longitudinale et une surface longitudinale ;
un ensemble d'électrodes à pointe segmentées (430 ; 530a, 530b, 530c, 730a, 730b ; 830a, 830b, 830c) qui sont disposées de manière circonférentielle autour de la pointe distale du corps de fil et qui sont électriquement isolées les unes des autres, chaque électrode à pointe segmentée de l'ensemble d'électrodes à pointe segmentées ayant une surface interne, une surface de stimulation externe opposée exposée le long de la surface longitudinale du corps de fil, et au moins un canal défini dans la surface interne et s'étendant le long d'au moins une partie d'une circonférence des électrodes à pointe segmentées, dans lequel une partie du corps de fil est disposée contre les surfaces internes de chacune des électrodes à pointe segmentées et de manière circonférentielle entre chacune des électrodes à pointe segmentées ;
au moins une électrode sans pointe disposée le long de la partie d'extrémité distale du corps de fil à proximité de l'ensemble d'électrodes à pointe segmentées ;
une pluralité de bornes disposées le long de la partie d'extrémité proximale du corps de fil ; et
une pluralité de conducteurs, chaque conducteur de la pluralité de conducteurs couplant électriquement la pluralité de bornes à chacune des électrodes à pointe segmentées et à la au moins une électrode sans pointe.

2. Fil de stimulation électrique implantable selon la revendication 1, dans lequel chaque électrode à pointe segmentée de l'ensemble d'électrodes à pointe segmentées a une section transversale en forme d'arc le long d'un axe parallèle à la longueur longitudinale du corps de fil au niveau de la pointe distale.

3. Fil de stimulation électrique implantable selon la revendication 1, dans lequel chaque électrode à pointe segmentée de l'ensemble d'électrodes à pointe segmentées a une section transversale en forme d'arc le long d'un axe transversal à la longueur longitudinale du corps de fil au niveau de la pointe distale.

4. Fil de stimulation électrique implantable selon la revendication 1, dans lequel chaque électrode à pointe segmentée de l'ensemble d'électrodes à pointe segmentées a une section transversale en forme d'arc le long de chacun parmi un axe parallèle à la longueur longitudinale du corps de fil au niveau de la pointe distale et un axe transversal par rapport à la longueur longitudinale du corps de fil au niveau de la pointe distale.

5. Fil de stimulation électrique implantable selon la revendication 1, dans lequel la surface de stimulation externe de chaque électrode à pointe segmentée de l'ensemble d'électrodes à pointe segmentées est de niveau avec la surface longitudinale du corps de fil.

6. Fil de stimulation électrique implantable selon la revendication 1, comprenant en outre une caractéristique de retenue de fil formée le long de la surface interne d'au moins une électrode à pointe segmentée de l'ensemble d'électrodes à pointe segmentées, la caractéristique de retenue de fil s'étendant plus profondément dans la surface interne de la au moins une électrode à pointe segmentée que les parties adjacentes de la surface interne.

7. Fil de stimulation électrique implantable selon la revendication 6, dans lequel la caractéristique de retenue de fil comprend une pluralité de rainures s'étendant le long de la surface interne de la au moins une électrode à pointe segmentée le long d'un axe longitudinal parallèle à la longueur longitudinale du corps de fil au niveau de la pointe distale.

8. Fil de stimulation électrique implantable selon la revendication 7, dans lequel le au moins un canal coupe au moins une partie d'au moins l'une de la pluralité de rainures.

9. Fil de stimulation électrique implantable selon la revendication 1, dans lequel la pluralité d'électrodes sans pointe comprend au moins un ensemble d'électrodes segmentées sans pointe ou au moins une électrode annulaire.

10. Système de simulation électrique comprenant :
un fil de stimulation électrique implantable selon la revendication 1 ;
un module de commande pouvant être couplé au fil de stimulation électrique implantable, le module de commande comprenant :
un boîtier, et
un sous-ensemble électronique disposé dans le boîtier ; et
un connecteur pour recevoir le fil de stimulation électrique implantable, le connecteur comprenant :
un boîtier de connecteur définissant un orifice, l'orifice étant configuré et agencé pour recevoir la partie d'extrémité proximale du corps de fil du fil de stimulation électrique implantable, et
une pluralité de contacts de connecteur disposés dans l'orifice, la pluralité de contacts de connecteur étant configurés et agencés pour se coupler à la pluralité de bornes disposées le long de la partie d'extrémité proximale du corps de fil lorsque la partie d'extrémité proximale du corps de fil est reçue par l'orifice.

11. Système de stimulation électrique selon la revendication 10, comprenant en outre une extension de fil couplant le fil de stimulation électrique implantable au module de commande.
